# EUROPEAN PATENT SPECIFICATION

(11) **EP 0 479 834 B1**
(45) Date of publication and mention of the grant of the patent: **10.11.1999**
(21) Application number: 90909585.3
(22) Date of filing: 29.06.1990
(51) Int. Cl.: C07K 16/44, C12P 21/08, G01N 33/53, G01N 33/532, G01N 33/531, G01N 33/543, C07F 9/64, A01N 57/14

(54) **COMPETITIVE ENZYME ASSAY FOR DETERMINATION OF PHOSPHOTHIOATE INSECTICIDES**
COMPETITIVES ENZYM-TESTVERFAHREN ZUM NACHWEIS VON PHOSPHOTHIOATEN
PROCEDE DE TEST IMMUNOENZYMATIQUE POUR DETERMINER LA PRESENCE DE PHOSPHOTHIOATE

(30) Priority: 30.06.1989 AU 501889
(43) Date of publication of application: 15.04.1992
(73) Proprietor: COMMONWEALTH SCIENTIFIC AND INDUSTRIAL RESEARCH ORGANISATION, Campbell, ACT 2612 (AU)
(72) Inventor: SKERRITT, John, Howard, Hornsby Heights, NSW 2077 (AU); HILL, Amanda, Susanne, Hornsby Heights, NSW 2077 (AU); McAdam, David, Peter, Duffy, ACT 2611 (AU)
(74) Representative: Perry, Robert Edward
(86) International application number: AU9000278
(87) International publication number: WO9100294

(56) References cited:
- EP-A- 0 044 441
- ACS SYMPOSIUM SERIES, vol. 451, 1990, pages 124-138, American Chemical Society, Washington, US; J.H. SKERRITT et al.: "Testing cereal products and samples by immunoassay", & INTERNATIONAL CHEMICAL CONGRESS OF PACIFIC BASIN SOCIETIES, Honolulu, Hawaii, 17th - 22nd December 1989
- JOURNAL OF AGRICULTURAL AND FOOD CHEMISTRY, vol. 29, 1981, pages 559-563, Washington, US; C.D. ERCEGOVICH et al.: "Development of a radioimmunoassay for parathian"
- Indian Journal of Medical Research, Vol 79, January 1984 (Calcutta, India) M.M. PADKI and M.B. BHIDE "Protective antibodies to fenitrooxon" pp 137-141. See pp 137-139.

## Description

The present invention relates to a competitive immunoassay for the determination of the phosphorothioate pesticides, chlorpyrifos-methyl, chlorpyrifos-ethyl pirimiphos-methyl and pyrimphos-ethyl. The invention particularly relates to a method of detecting these organophosphorus pesticides in water, foods and agricultural produce (especially grain).

Organophosphorus pesticides are a large class of insecticides which function by inhibition of cholinesterase in the insect neuromuscular junction. Such inhibition produces paralysis, suffocation and eventually death.

Organophosphates are routinely applied to grain and other produce intended for storage for several months or for export before further processing or consumption. As insect infestation will render the product unsuitable for human consumption or sale, it is important to ensure that application of organophosphorus protectant has been evenly distributed, and in sufficient quantities.

It is critical to ensure that levels of organophosphates in grain and other produce for human consumption do not exceed maximum residue levels (MRL) set by appropriate legislation. For example, the MRL for chlorpyrifos-methyl, at least in the USA, in wheat grain is 10 ppm, in white flour 2 ppm and in white bread 0.5 ppm.

While reliable methods exist for the specific determination of organophosphates by chromatographic methods, these methods are not suitable for on-farm or atmarket testing, or for use by small or less well-equipped food, grain or produce laboratories, or for use in the field.

Conventional methods for detection or determination of organophosphorus pesticides are accurate and precise, but are not well suited to either field analysis of the compound or high-throughput analysis in small laboratories with minimal equipment. Analysis by gas-liquid chromatography or high-performance liquid chromatography suffers from high equipment cost, need for skilled operators or analysts and both low sample throughput and relatively long sample analysis time. Removal of interfering components (sample "clean-up") is needed for most chromatographic analyses. Thin-layer chromatography does not require high-cost equipment, but is only semiquantitative and the organophosphorus pesticide present can be difficult to distinguish from related compounds.

Enzymic assays for organophosphates based on cholinesterase inhibition are fast and relatively simple, but are not specific for individual organophosphates. In some cases, sensitivity can be insufficient.

Immunoassays, based on either polyclonal or monoclonal antibodies, are a useful alternative to conventional chemical and instrumental assays for organophosphorus pesticides. Small organic molecules are not by themselves immunogenic, but can be made immunogenic following chemical conjugation to a suitable carrier such as a protein. The site and nature of conjugation and carrier protein can affect the specificity and affinity of antibodies produced, in a manner that cannot fully be predicted by those skilled in the art. For example, preparation of reduced parathion (a compound closely related to the hapten used in the present invention) coupled with long bridging groups (glutarimine and diazobenzoic acid) did not yield conjugates producing antisera with useful reaction with parathion (Vallejo et al, J. Agric. Food Chem. 30 (1982) 572).

In recent years, techniques of producing monoclonal antibodies have been developed which make it possible to obtain homogeneous, highly specific antibodies. Generally, such antibodies are produced by immunizing an animal with a protein, obtaining antibody-producing cells from the animal, and fusing the antibody-producing cells with strains of myeloma cells, i.e., tumour cells, to produce hybridomas which are isolated and cultured as monoclones. The monoclonal hybridomas may either be cultured in vitro or may be grown as tumours in a host animal. Since each antibody-producing cell produces a single unique antibody, the monoclonal cultures of hybridomas each produce a homogeneous antibody which may be obtained either from the culture medium of hybridoma cultures grown in vitro or from the cells, ascitic fluid, or serum of a tumour-bearing host animal.

Not all of the hybridoma clones which result from fusing myeloma cells with antibody-producing cells are specific for the desired pesticide (or for functional groups upon the pesticide characteristic of that class of molecules), since many of the hybridomas will make antibodies which the inoculated animal has produced to react with other foreign substances. Even antibodies against the subject antigen will differ from clone to clone, since antibodies produced by different cells may react with different antigenic determinants of the same molecule. From each clone, therefore, it is necessary to obtain the resulting antibody, to test its reactivity with the subject pesticide, and to test its specificity by determining which particular organophosphorus pesticides it recognizes. Further, only certain antibodies or antisera function in specific immunoassay configurations or formats.

Immunochemical methods to detect or to determine organophosphorus cholinesterase inhibitors, including pesticides, are known. These include polyclonal antisera to malathion (Haas et al, Proc. Soc. Exp. Biol. Med. 129 (1968) 546; Centeno et *al,* Int. Arch. Allergy 37 (1970)) parathion (Ercegovich et *al,* J. Agric. Food Chem. 29 (1981) 559; Ngeh-Ngwainbi et *al,* J. Amer. Chem. Soc. 108 (1986) 5444) and paraoxon (Hunter et *al,* Life Sci. 30 (1982) 355; Lober et *al,* Acta. Biol. Med. Ger. 41 (1982) 487; Heldman et *al,* FEBS Lett. 180 (1985) 243) and monoclonal antibodies to paraoxon (Brimfield et *al,* J. Agric. Food Chem. 33 (1985) 1237) and soman (Hunter et *al,* FEBS Lett. 149 (1982) 147; Buenafe et *al,* Mol. Immunol. 24, (1987) 401). Cross-reaction of these antibodies with organophosphates such as chlorpyrifos-methyl, chlorpyrifos-ethyl and pirimiphos-methyl was not normally assessed, nor would they be specific for these organophosphates. Immunoassays have also been developed which specifically detect organophosphate pesticides with diethyl thiophosphate esters but these did not detect chlorpyrifos-methyl (Suedi et al, Kiel. Melchwirtsch. Forschungsber 40 (1986) 179).

Heldman et *al, supra,* disclose the derivatization of an organophosphate pesticide by reaction of a beta-alanine derivative with the organophosphate phosphorus for conjugation with a carrier protein for the purpose of preparing antibodies to the organophosphate pesticide paroxon. A trimethylsilyl ester of beta-alanine was used in the nucleophilic substitution reaction on O-(4-nitrophenyl)-O-methyl phosphorochloridate. Such a derivative is unstable to moisture.

According to a first aspect of the present invention, a competitive immunoassay method, for the quantification or detection of a phosphorothioate selected from chlorpyrifos-methyl, chlorpyrifos-ethyl, pirimphos-methyl and pirimphosethyl, in a produce, grain, food or environmental sample, comprises the steps of:
(a) contacting the sample with an antibody or fragment thereof which binds with high specificity to the phosphorothioate, in the presence of a known amount of an enzyme-labelled conjugate of the phosphorothioate, wherein the enzyme is attached through a carboxylic acid group of an ester or acid spacer on the phosphorothioate group of the labelled phosphorothioate; and
(b) determining the binding of the labelled phosphorothioate to the antibody or antibody fragment, the binding being inversely proportional to the amount of the phosphorothioate in the sample;
wherein the antibody is recoverable from an animal immunised with an immunogen conjugate of an immunogenic macromolecule and phosphorothioate as defined above, wherein the macromolecule is attached through a carboxylic acid group of an ester or acid spacer on the phosphorothioate group of the phosphorothioate.

According to a second aspect of the invention, a kit comprises the antibody or fragment defined above, and a known amount of the enzyme-labelled conjugate.

In particular, kits may comprise a packet containing microwell plates coated with antibody specific for organophosphates, or strips or other suitable solid support, a standard solution or solutions or preparation containing a defined amount of phosphorothioate, an enzyme-conjugated derivative of phosphorothioate, an enzyme substrate, washing and colour development stopping solutions, and non-specific binding blocking reagents.

Alternatively, kits may comprise a packet containing phosphorothioate derivative-conjugated macromolecule-coated microwell plates or strips or other suitable solid support, a standard solution or solutions or preparation containing a defined amount of phosphorothioate, antibody to phosphorothioate, washing and colour development stopping solutions, and non-specific binding blocking reagents.

Alternatively, kits to detect organophosphate may be constructed to rely on the principle of agglutination, adherence, fluorescence, visual immunoassay, radioimmunoassay or chemiluminescence.

The antibodies or fragments thereof that are used in the present invention, may be polyclonal. However, monoclonal antibodies and fragments are more preferred.

The conjugate used in the present invention may be prepared by chemically activating the organophosphorus pesticide by means of derivatization through a phosphorus atom, by means of a protected ester or acid of a suitable spacer-arm compound. Preferably the spacer is beta-alanine. The acid reactive group on the spacer may be protected with any protective groups which can be removed in non-basic conditions. Suitable protective groups include Trityl (triphenylmethyl), t-pentyl, t-hexyl and trimethylsilyl ethyl; t-butyl is particularly preferred. The organophosphate conjugate derivatives may be subsequently rendered chemically reactive with carrier macromolecules by mild and facile hydrolysis.

The antibody or fragment used in the present invention is preferably bound to a support. In the method of the invention, a solution of suitably labelled antibody specific for the phosphorothioate or a solution of suitably labelled 0, O-dialkyl, O- phosphorothioate (hapten) is applied to the support and, optionally after washing, the labelled antibody or hapten bound to this solid phase support is subjected to an appropriate signal amplification step or other detection procedure.

An appropriate signal amplification step may be an enzyme immunoassay step, where an appropriate enzyme may be coupled to the antibody and subsequently substrate is added to the antigen/enzyme-labelled antibody complex. Alternatively radioimmunoassay, fluorescence immunoassay, chemiluminesence, agglutination or adherence electrochemical or optical methods or combinations thereof may be used as appropriate detection steps.

Suitable solid phase supports may have a variety of configurations and may include tubes, well plates, microplates, elongate sticks or thin strips or beads. The materials that such solid phase surfaces may be formed from include polystyrene or other suitable plastics, nitrocellulose, nylon, polyvinylidene difluoride, glass, silica or other suitable material, such material having been precoated with either polyclonal or monoclonal antibody specific for the O,O-dialkyl -O- phosphorothioate or a suitable carrier macromolecule substituted with the organophosphate.

Hybridoma cell lines from which antibodies for use in this invention are available, are preferably produced by the fusion of an antibody producing cell and a myeloma cell derived from a murine species. The antibody producing cells are preferably spleen cells. Any suitable myeloma cell line may be used. However,it is desirable to use a well characterised cell line of which a number are in common usage.

The testing method according to the present invention does away with the need for sample "clean-up" by solvent extraction, phase transfer or adsorption chromatography (methods commonly used in sample preparation for chromatographic pesticide analysis). The procedure is adaptable to either rapid (under 15 minutes) analysis of individual samples or simultaneous analysis of many dozen samples, does not require expensive automated equipment, and allows such assays to be performed by small laboratories or in field or market situations. Further, the present invention is suitable for raw grain or produce or cooked or processed foods; the organophosphate may be extracted in a single step. The method of the present invention is simple, inexpensive and reliable and allows either a qualitative or quantitative determination of organophosphate. Additionally the sensitivity of the test may be readily altered by changing antibody or antigen concentration, hapten substitution ratios or solvent to solid extraction ratios.

The invention is herein further described by way of examples describing preparation of the immunogens, polyclonal antisera, cell-lines and monoclonal antibodies according to the present invention and the use of those antibodies to determine organophosphates in foodstuffs and agricultural produce such as grain.

### Production of immunogen conjugates

Thiophosphoryl chloride was refluxed in methanol/trichloromethane, then the product reacted under alkaline conditions with t-butyl beta-alanine ester to give the bifunctional reagent (IIa) which was followed by reaction with 3,5,6-trichloro-2-pyridinol (B) or 2-diethylamino-4-hydroxy-6-methylpyrimidine (C) in acetonitrile to yield (IIB) or (IIC).

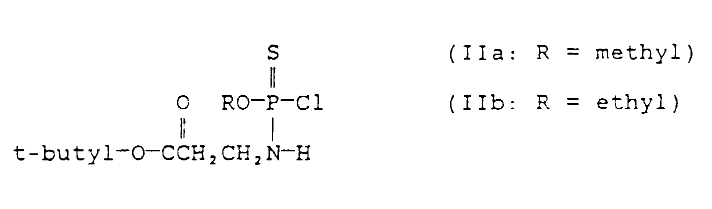

Thiophosphoryl chloride may also be refluxed in ethanol/trichloromethane and then the product reacted as above with t-butyl beta-alanine ester to give (IIb) followed by reaction with, for example, 3,5,6-trichloro-2-pyridinol (B) to yield (IID).

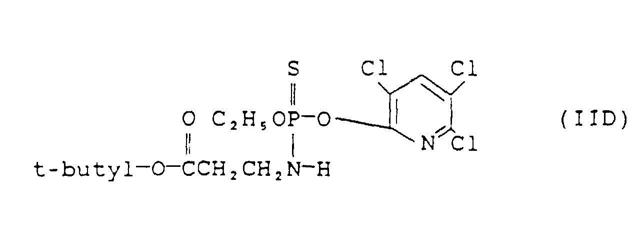

The t-butyl esters (II A-D) are then hydrolyzed and reacted with N-hydroxysuccinimide in dicyclohexyldicarboxinimide (DCC)/methylene chloride to yield III B-D. Theses compounds (III B-D) were reacted with the various carrier proteins, including chicken IgG, hen egg ovalbumin and keyhole limpet haemocyanin to yield immunogens. The degree of coupling (hapten-to-carrier molar ratio) was between 2 and 50 for each conjugate and could be varied by altering molar ratios of reactants or the reaction conditions.

The derivatization of an organophosphate pesticide by reaction of a beta-alanine derivative with the organophosphate phosphorus for conjugation with a carrier protein for the purpose of preparing antibodies to the organophosphate pesticide, paroxon, is disclosed by Heldman, E. Balan, A. Horowitz, D. Ben-Zion, S. and Torten, M. FEBS Letters, 180 (1985), 243-247. However, these workers derivatized one particular organophosphate pesticide only and used a trimethyl silyl ester of beta-alanine in the nucleophilic substitution reaction on O-(4-nitrophenyl)-O-methyl phosphorochloridate. Such a derivative is unstable to moisture and since it cannot be used as a general method, a new synthesis is required for each organophosphate. By preparing the bifunctional reagent (II), an easier and more useful method of conjugation is obtained, because it may be used for any O,O-diakyl-O-phosphorothioate and the derivatives can be stored for extended periods prior to coupling reactions with macromolecules.

It will be appreciated by those skilled in the art that other conjugation methods, bifunctional reagents and carrier proteins may yield immunogens producing antibodies with reactivity for fenitrothion and closely related organophosphates.

### Production of polyclonal antisera and monoclonal antibodies

Individual rabbits (New Zealand White) were immunized with one of the immunogen conjugates. The initial immunization in 50% Freunds complete adjuvant (1000 µg conjugate, half subcutaneous, half intramuscular)) was followed by 500 µg conjugate per rabbit in Freunds incomplete adjuvant, two and four weeks later and at monthly intervals thereafter. Antibodies were harvested from serum using Protein A-Sepharose chromatography.

For preparation of monoclonal antibodies mice (Balb/c) were injected with one of the immunogen conjugates. The initial immunization in 50% Freunds complete adjuvant (400 µg conjugate) was followed by 100 µg conjugate per mouse in Freunds incomplete adjuvant, two and four weeks later. Doses were divided with half given subcutaneously and half intramuscularly. Four to eight weeks later mice were given an intraperitoneal booster injection (900 µg) and the spleens removed for fusion, four days later. Spleen cells were fused with an appropriate mouse myeloma cell line (Sp 2/0, Galfre et al, Nature 266 (1977) 550) using polyethyleneglycol. The details of the fusion protocol and culture techniques have been described elsewhere (Shulman et al, Nature 276 (1978) 269). Fused cells (hybridomas) were separated by dilution and antibody-secreting clones were subcloned by limiting dilution. For some subclones, hybridomas were grown as ascites tumours for 10 days in mice.

### Preparation of enzyme-labelled phosphorothioate

Compounds (IIIB-D) were coupled to horseradish peroxidase by means of the beta-alanine spacer arm, following deprotection with aqueous trifluoroacetic acid and treatment with acidified methanol. The resultant amine was reacted with periodate-treated horseradish peroxidase at pH 9.5 for hours at 20°C. Unreacted aldehyde groups on the enzyme were quenched with ethanolamine.

### Assay Format 1.

The solid phase is coated with phosphorothioate-specific antibody. Preferentially the antibody is coated at 10µg/mL in 50mM sodium carbonate buffer, pH 9.6. Non-specific binding of immunoreactants is then minimized by treatment with a suitable protein or detergent, for example, 10mg/mL bovine serum albumin in 50mM sodium phosphate, pH 7.2 - 0.9% sodium chloride. The grain or food or other sample to be analyzed is extracted in neat methanol or ethanol, although other polar water-miscible solvents may be used; such as isopropanol, acetonitrile and acetone. Mixtures of water and these solvents may also be used. The grain, food or other extract is diluted five-fold in a protein and detergent-containing buffer (for example 10mg/mL bovine serum albumin or lmg/mL Teleostean fish skin gelatin in 0.05% (v/v) Tween in phosphate-buffered saline) applied to the solid phase and enzyme-labelled phosphorothioate (diluted similarly) is added immediately. A standard is prepared in methanol or ethanol and dilutions of this standard assayed simultaneously.

An incubation time of 30 min with chlorpyrifos-methyl (P9) or pirimiphos-methyl (P11) conjugated to horseradish peroxidase was used for the results shown in Table 1, together with 10 min chromogenic substrate (2,2'-azino-bis (3-ethyl benzthiazoline -6- sulfonic acid)) incubation time. The chromogen 3,3',5,5'-tetramethylbenzidine may also be used.

Analysis by this technique is based on the competition between phosphorothioate in the test sample and enzyme-labelled phosphorothioate for binding to phosphorothioate-specific antibody on the solid phase. There is a decrease in the binding of the enzyme-labelled phosphorothioate as the concentration of phosphorothioate increases in the test sample; this is manifest in decreasing absorbance of the coloured enzyme product. The concentration of phosphorothioate in an unknown sample may be determined by comparing the degree of inhibition of antibody binding caused by the addition of the sample extract with that resulting from the addition of known amounts of phosphorothioate. For analysis of extracts of most foods, grain, agricultural produce and water, no clean-up of the extracts was necessary.

Specificity properties of the antibodies and some results obtained with grain samples are shown in Table 1. The sensitivities obtained are sufficient for analysis of phosphorothioate both at low levels of addition and at levels near or in excess of specified maximum residue levels. In addition, the assay may be used to determine residues of organophosphates in produce stored for long periods or following processing.

### Assay format 2.

The solid phase is coated with a pesticide-macromolecule conjugate. For the results listed in Table 1, the immunogen conjugate of chlorpyrifos-methyl (F7) was used. Following pretreatment to minimize non-specific binding of conjugate by treatment with a suitable protein or detergent, the sample to be analyzed is added. The grain, food or other sample may either be extracted in methanol, ethanol or other polar water-miscible solvent, or otherwise be extracted with a volatile organic solvent such as dichloromethane or acetone. The solvent may be removed by evaporation and the residue redissolved in a smaller volume of methanol. The extract or reconstituted residue is diluted in a protein and detergent - containing buffer and applied to the solid phase and antibody is added immediately. A standard is prepared in methanol and dilutions of this standard assayed simultaneously. Such antibody may either be enzyme-labelled by direct covalent association or a suitable amplification step used to detect binding of the antibody. Analysis by this technique is based on the competition between phosphorothioate in the test sample and solid phase- bound phosphorothioate, for binding to antibody in solution. There is a decrease in the binding of antibody as the concentration of organophosphate increases in the test sample; this is manifest in decreasing absorbance of the coloured enzyme product, in this example. The concentration of organophosphate may be assessed as described in assay format 1. Clean-up of the extracts was not necessary.

An antibody incubation time of 30 minutes was used, together with 20 min (2,2'-azino bis (3-ethylbenzthiazoline-6-sulfonic acid)) substrate incubation time. The overall assay time can be reduced to 5-10 minutes or increased to 18 hours for batchwise analysis of large numbers of (several hundred) samples. In the latter case, sensitivity to phosphorothioate is also increased.

Sensitivity can be increased even further by use of volatile organic solvents for extraction of the sample, evaporation of solvent and redissolving the residue in a smaller volume of water-miscible organic solvent.

**Table 1.**

| Antibody | F9 | F7 | P11 |
|---|---|---|---|
| Assay Format | 1 | 2 | 1 |
| Control Pesticide | CP | CP | PY |
| | | | |

| Compound | | | |
|---|---|---|---|
| | | | |
| fenitrothion (FN) | - | 0.01 | - |
| methylparathion | - | 0.003 | - |
| dicapthon | - | 0.01 | - |
| chlorpyrifos-methyl (CP) | 1.0 | 1.0 | - |
| pirimiphos-methyl (PY) | - | - | 1.0 |
| chlorpyrifos-ethyl | - | 0.07 | - |
| pirimiphos-ethyl | - | - | 2.9 |

The data shown in Table 1 are potencies relative to the control pesticide (determined as relative concentration yielding 50% inhibition of antibody binding). Absolute amounts of control pesticide yielding 50% inhibition of antibody binding in competition ELISA are: P9 (0.001 ng), F7 (50 ng), Pll (2 ng).

## Claims

1. A competitive immunoassay method, for the quantification or detection of a phosphorothioate selected from chlorpyrifos-methyl, chlorpyrifos-ethyl, pirimphos-methyl and pirimphos-ethyl, in a produce, grain, food or environmental sample, comprising the steps of:
(a) contacting the sample with an antibody or fragment thereof which binds with high specificity to the phosphorothioate, in the presence of a known amount of an enzyme-labelled conjugate of the phosphorothioate, wherein the enzyme is attached through a carboxylic acid group of an ester or acid spacer on the phosphorothioate group of the labelled phosphorothioate; and
(b) determining the binding of the labelled phosphorothioate to the antibody or antibody fragment, the binding being inversely proportional to the amount of the phosphorothioate in the sample;
wherein the antibody is as recoverable from an animal immunised with an immunogen conjugate of an immunogenic macromolecule and a phosphorothioate as defined above, wherein the macromolecule is attached through a carboxylic acid group of an ester or acid spacer on the phosphorothioate group of the phosphorothioate.

2. A method according to claim 1, wherein the spacer is beta-alanine.

3. A method according to claim 1 or claim 2, wherein the antibody is a monoclonal antibody.

4. A method according to claim 1 or claim 2, wherein the antibody is a polyclonal antibody.

5. A method according to any preceding claim, wherein the enzyme is horseradish peroxidase.

6. A method according to any preceding claim, wherein the antibody or fragment thereof is bound to a support.

7. A kit for the quantification or detection of a phosphorothioate as defined in claim 1, comprising:
(a) an antibody or fragment thereof as defined in claim 1; and
(b) a known amount of an enzyme-labelled conjugate as defined in claim 1.

8. A kit as claimed in claim 7, wherein the spacer is beta-alanine.

9. A kit as claimed in claim 7 or claim 8, wherein the antibody is a monoclonal antibody.

10. A kit as claimed in claim 7 or claim 8, wherein the antibody is a polyclonal antibody.

11. A kit as claimed in any of claims 7 to 10, wherein the enzyme is horseradish peroxidase.

12. A kit as claimed in any of claims 7 to 11, wherein the antibody or fragment thereof is bound to a solid support.

13. A kit as claimed in any of claims 7 to 12, further comprising a standard solution or preparation containing a known amount of the phosphorothioate.

## Patentansprüche

1. Competitives Immunoassay-Testverfahren zur Quantifizierung oder Bestimmung eines Phosphorothioats, ausgewählt aus Chlorpyrifos-Methyl, Chlorpyrifos-Ethyl, Pirimphos-Methyl und Pirimphos-Ethyl, in einem Erzeugnis, einem Getreide, einem Nahrungsmittel oder einer Umweltprobe, das folgende Schritte aufweist:
a) Inkontaktbringen der Probe mit einem Antikörper oder dessen Fragment, welcher mit hoher Spezifität an das Phosphorothioat bindet, im Beisein einer bekannten Menge eines enzymmarkierten Konjugats des Phosphorothioats, wobei das Enzym über eine Carbonsäuregruppe eines Ester- oder Säurespacers an die Phosphorothioatgruppe des markierten Phosphorothioats gebunden ist; und
b) Bestimmung der Bindung des markierten Phosphorothioats an den Antikörper oder das Antiköperfragment, wobei die Bindung umgekehrt proportional zu der Menge des Phosphorothioats in der Probe ist;
wobei der Antikörper wie erhältlich aus einem mit einem Immunkonjugat eines immunogenen Makromoleküls und einem Phosphorothioat, wie oben definiert, immunisierten Tier ist und wobei das Makromolekül über eine Carbonsäuregruppe eines Ester- oder Säurespacers an die Phosphorothioatgruppe des Phosphorothioats gebunden ist.

2. Verfahren nach Anspruch 1, wobei der Spacer ein beta-Alanin ist.

3. Verfahren nach Anspruch 1 oder Anspruch 2, wobei der Antikörper ein monoklonaler Antikörper ist.

4. Verfahren nach Anspruch 1 oder Anspruch 2, wobei der Antikörper ein polyklonaler Antikörper ist.

5. Verfahren nach einem der vorausgehenden Ansprüche, wobei das Enzym Meerrettich-Peroxidase ist.

6. Verfahren nach einem der vorausgegangenen Ansprüche, wobei der Antikörper oder das Fragment davon an einen Träger gebunden ist.

7. Kit für die Quantifizierung oder den Nachweis eines Phosphorothioats, wie in Anspruch 1 definiert, enthaltend:
(a) einen Antikörper oder ein Fragment davon, wie in Anspruch 1 definiert; und
(b) eine bekannte Menge eines enzymmarkierten Konjugats, wie in Anspruch 1 definiert.

8. Kit nach Anspruch 7, wobei der Spacer beta-Alanin ist.

9. Kit nach Anspruch 7 oder Anspruch 8, wobei der Antikörper ein monoklonaler Antikörper ist.

10. Kit nach Anspruch 7 oder Anspruch 8, wobei der Antikörper ein polyklonaler Antikörper ist.

11. Kit nach einem der Ansprüche 7 bis 10, wobei das Enzym Meerrettich-Peroxidase ist.

12. Kit nach einem der Ansprüche 7 bis 11, wobei der Antikörper oder das Fragment davon an einen festen Träger gebunden ist.

13. Kit nach einem der Ansprüche 7 bis 12, weiter enthaltend eine Standardlösung oder -zubereitung, die eine bekannte Menge des Phosphorothioats enthält.

## Revendications

1. Procédé d'analyse immunologique compétitive pour la quantification ou la détection d'un phosphorothioate choisi parmi le chlorpyrifos-méthyle, le chlorpyrifos-éthyle, le pirimphos-méthyle et le pirimphos-éthyle dans un échantillon de produit, de grains ou d'aliment ou dans un échantillon environnemental, comprenant les étapes selon lesquelles:
(a) on met en contact l'échantillon avec un anticorps ou un de ses fragments qui se lie avec une spécificité élevée au phosphorothioate, en présence d'une quantité connue d'un conjugué du phosphorothioate marqué par une enzyme, l'enzyme étant liée au groupe phosphorothioate du phosphorothioate marqué par l'intermédiaire d'un groupe acide carboxylique d'un espaceur ester ou acide ; et
(b) on détermine la liaison du phosphorothioate marqué à l'anticorps ou au fragment d'anticorps, la liaison étant inversement proportionnelle à la quantité de phosphorothioate dans l'échantillon ;
dans lequel l'anticorps est un anticorps pouvant être récupéré à partir d'un animal immunisé avec un conjugué immunogène d'une macromolécule immunogène et d'un phosphorothioate tel que défini ci-dessus, la macromolécule étant liée au groupe phosphorothioate du phosphorothioate par l'intermédiaire d'un groupe acide carboxylique d'un espaceur ester ou acide.

2. Procédé selon la revendication 1, dans lequel l'espaceur est la β-alanine.

3. Procédé selon la revendication 1 ou la revendication 2, dans lequel l'anticorps est un anticorps monoclonal.

4. Procédé selon la revendication 1 ou la revendication 2, dans lequel l'anticorps est un anticorps polyclonal.

5. Procédé selon l'une quelconque des revendications précédentes, dans lequel l'enzyme est la peroxydase de raifort.

6. Procédé selon l'une quelconque des revendications précédentes, dans lequel l'anticorps ou son fragment est lié à un support.

7. Trousse pour le dosage ou la détection d'un phosphorothioate selon la revendication 1, comprenant :
(a) un anticorps ou un de ses fragments tel que défini dans la revendication 1; et
(b) une quantité connue d'un conjugué marqué par une enzyme tel que défini dans la revendication 1.

8. Trousse selon la revendication 7, dans laquelle l'espaceur est la β-alanine.

9. Trousse selon la revendication 7 ou la revendication 8, dans laquelle l'anticorps est un anticorps monoclonal.

10. Trousse selon la revendication 7 ou la revendication 8, dans laquelle l'anticorps est un anticorps polyclonal.

11. Trousse selon l'une quelconque des revendications 7 à 10, dans laquelle l'enzyme est la peroxydase de raifort.

12. Trousse selon l'une quelconque des revendications 7 à 11, dans laquelle l'anticorps ou son fragment est lié à un support solide.

13. Trousse selon l'une quelconque des revendications 7 à 12, comprenant en outre une solution ou une préparation étalon contenant une quantité connue du phosphorothioate.
